(19) Europäisches Patentamt
European Patent Office
Office européen des brevets

(11) Publication number : 0 282 556 B1

(12) # EUROPEAN PATENT SPECIFICATION

(45) Date of publication of patent specification : **22.04.92 Bulletin 92/17**

(51) Int. Cl.$^5$ : **C07D 233/72**

(21) Application number : **87906238.8**

(22) Date of filing : **25.08.87**

(86) International application number : **PCT/US87/02067**

(87) International publication number : **WO 88/01999 24.03.88 Gazette 88/07**

(54) **PROCESS FOR THE PREPARATION OF 3-(1-HYDROXYALKYL)-5,5-DIPHENYLHYDANTOIN.**

The file contains technical information submitted after the application was filed and not included in this specification

(30) Priority : **19.09.86 US 909140**

(43) Date of publication of application : **21.09.88 Bulletin 88/38**

(45) Publication of the grant of the patent : **22.04.92 Bulletin 92/17**

(84) Designated Contracting States : **DE FR GB IT**

(56) References cited :
**US-A- 4 163 058**
**Zaugg and Martin, Organic Reactions, vol. 14, 1965, pp. 107-108**
**J. March, Advanced Organic Chemistry, 1968, pp. 667-668**

(73) Proprietor : **THE DU PONT MERCK PHARMACEUTICAL COMPANY**
**Barley Mill Plaza, Building 25**
**Wilmington, Delaware 19880 (US)**

(72) Inventor : **MAI, Khuong, H. X.**
**108 South Orchard**
**Waukegan, IL 60085 (US)**
Inventor : **PATIL, Ghanshyam**
**340 Albert Drive**
**Vernon Hills, IL 60061 (US)**

(74) Representative : **Seaborn, George Stephen et al**
**c/o Edward Evans & Co. Chancery House**
**53-64 Chancery Lane**
**London WC2A 1SD (GB)**

EP 0 282 556 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

The present invention relates to a novel process fot the preparation of 3-(1-hydroxyalkyl)-5,5-diphenylhydantoins or simply 3-hydroxyalkylphenytoins. Such compounds are very important intermediates in the preparation of phenytoin prodrug. U.S. Patent No. 4,163,058 describes the preparation of such intermediates. The given procedure calls for large amounts of solvent, e.g., water, to solubilize the reactants. The low solubility of phenytoin in water creates an immediate inconvenience due to large volume of solvent and prolonged reaction period. Zaugg and Martin, Org. Reactions, 14, 52-264 (1985), pages 107-108 discloses the reaction of dichloroacetaldehyde with urea, which is the basis of the preparation of several hundred compounds of this class.

### SUMMARY OF THE INVENTION

In accordance with the present invention, there is provided a process of preparing a 3-(1-hydroxyalkyl)-5,5-diphenylhydantoin of the formula

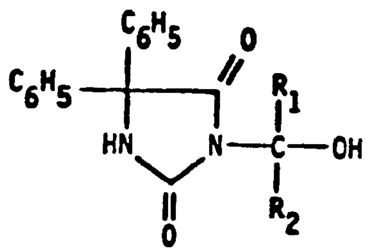

wherein $R_1$ and $R_2$ are each independently hydrogen, $C_1$-$C_6$ alkyl, or $R_1$ and $R_2$ together with the carbon atom to which they are attached form a 3 to 12 member cycloalkylene group, which process comprises: mixing in a single reaction vessel an aldehyde or ketone of formula $R_1R_2C{=}O$, a solvent, a catalytic amount of an inorganic strong base, e.g. NaoH, and phenytoin, the phenytoin being added to the vessel after the aldehyde or ketone, the solvent and the base have been added to the vessel, to produce the 2-(1-hydroxyalkyl)-5,5-diphenylhydantoin by reaction of the aldehyde or ketone and the phenytoin, characterized in that the solvent is an alcohol.

The process of the invention can be depicted by the following reaction scheme:

30 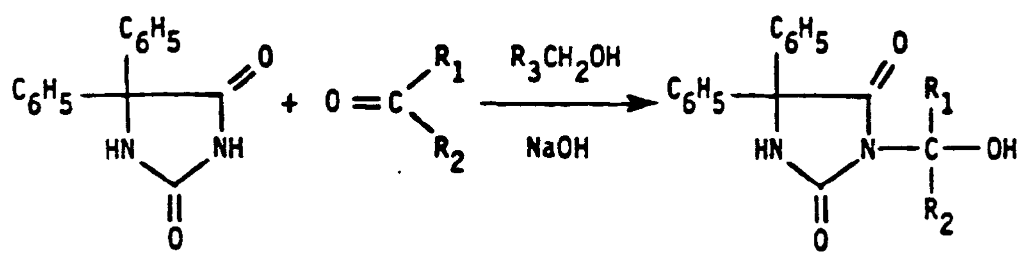


In the above reaction scheme, $R_1$, $R_2$ and $R_3$ are each independently hydrogen, $C_1$-$C_6$ alkyl, or $R_1$ and $R_2$ together with the carbon atom form a 3 to 12 member cycloalkyl group.

The term $C_1$-$C_6$ alkyl as used herein refers to straight or branched chain alkyl radicals containing from 1 to 6 carbon atom including methyl, ethyl, n-propyl, isopropyl, n-butyl, sec-butyl, tert-butyl, n-pentyl, iso-pentyl, sec-pentyl, tert-pentyl and neo-pentyl.

The term "cycloalkylene" as used herein refers to cyclic saturated aliphatic radicals containing 3 to 12 carbon atoms in the ring, such as cyclopropyl, cyclobutyl, cyclopentyl or cyclododecyl.

In the process, phenytoin is added to a solution of an aldehyde or a ketone in an alcohol in the presence of catalytic amount of strong base, e.g. NaoH. A suitable temperature is 20°C to about 100°C, preferably ambient temperature. A suitable reaction time is from 1 minute to 2 hours, preferably 15 minutes. Suitable solvents are methanol, ethanol, n-propanol, isopropanol, butanol, or a mixture thereof.

In the described method, phenytoin and the carbonyl compound may be theoretically used in an amount

of about one equivalent each for the purpose of preparing the 2-hydroxyalkylphenytoin but it is preferable to use an excess amount of the carbonyl compound which functions both as reactant and as additional solvent.

DETAILED DESCRIPTION OF THE INVENTION

In accordance with one embodiment of the present invention, the preparation of a 3-hydroxyalkylphenytoin was conducted as follows:

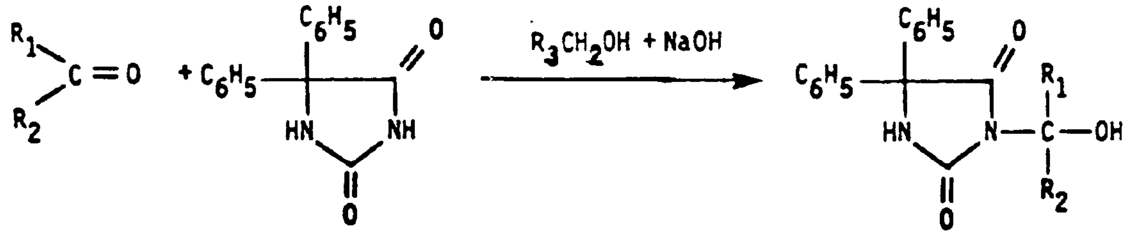


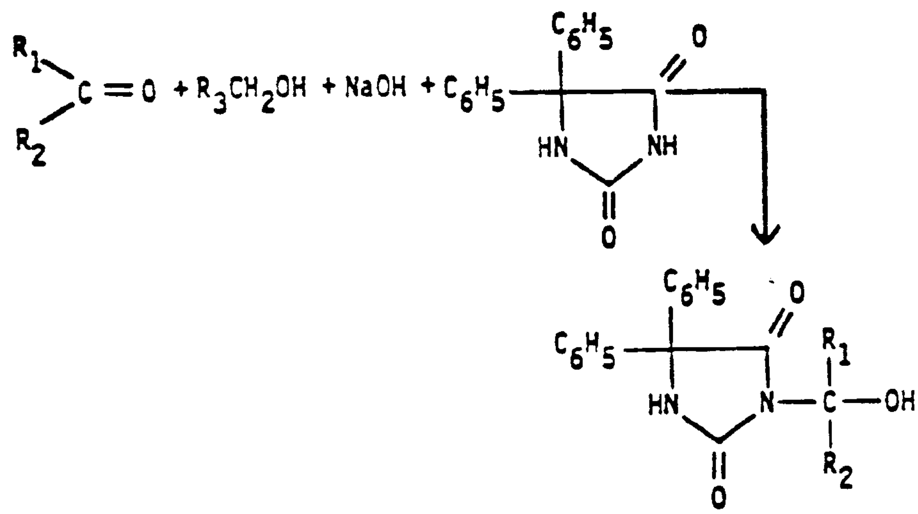

A general procedure is as follows: One (1) kg of phenytoin in one lot was added to a stirred mixture of 1 liter (L) of 37% aqueous formaldehyde, 1 L of ethyl alcohol and 5 g of sodium hydroxide. At the end of the addition, the solution became homogeneous. After one minute, white granulars of 2-hydroxymethylphenytoin started to separate. Stirring was continued for another 15 minutes. Water (1 L) was added and the product filtered, washed with water and air-dried. Yield: 1.01 kg (90%), melting point 184-186°C.

The process offers the advantage of significantly reduced amount of required solvent in comparison to the process of the prior art and thus provides a convenient, simple and fast procedure for the preparation of 2-hydroxyalkylphenytoin.

## Claims

1. A process of preparing a 3-(1-hydroxyalkyl)-5,5-diphenylhydantoin of the formula

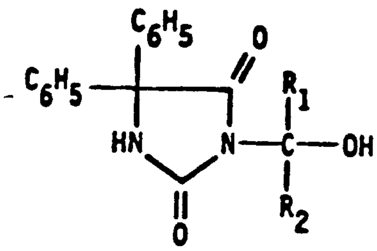

wherein $R_1$ and $R_2$ are each independently hydrogen, $C_1$-$C_6$ alkyl, or $R_1$ and $R_2$ together with the carbon atom to which they are attached form a 3 to 12 member cycloalkylene group, which process comprises: mixing in a single reaction vessel an aldehyde or ketone of formula $R_1R_2C=O$, a solvent, a catalytic amount of an inorganic strong base, and phenytoin, the phenytoin being added to the vessel after the aldehyde or ketone, the solvent and the base have been added to the vessel, to produce the 3-(1-hydroxyalkyl)-5,5-diphenylhydantoin by reaction of the aldehyde or ketone and the phenytoin, characterized in that the solvent is an alcohol.

2. The process of claim 1 wherein the reaction is carried out at a temperature from about 20°C to 100°C.

3. The process of claim 2, wherein the reaction is conducted at ambient temperature.

4. The process of any preceding claim, wherein the reaction is conducted for a time of from about 1 minute to about 2 hours.

5. The process of claim 4, wherein the process is conducted for a time of about 15 minutes.

6. The process of any preceding claim wherein the solvent is selected from methanol, ethanol, propanol, butanol, and mixtures thereof.

## Patentansprüche

1. Verfahren zur Herstellung eines 3-(1-Hydroxyalkyl)-5,5-diphenylhydantoins der Formel

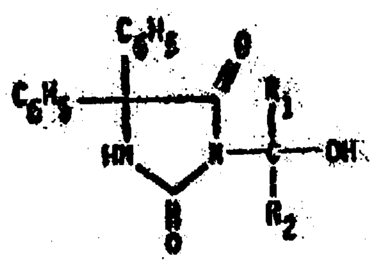

worin $R_1$ und $R_2$ jeweils unabhängig Wasserstoff, $C_1$-$C_6$-Alkyl sind, oder $R_1$ und $R_2$ zusammen mit dem Kohlenstoff-Atom, an welches sie geknüpft sind, eine 3- bis 12gliedrige Cycloalkylen-Gruppe bilden, umfassend das Vermischen eines Aldehyds oder Ketons der Formel $R_1R_2C=O$, eines Lösungsmittels, einer katalytischen Menge einer anorganischen starken Base und Phenytoin in einem einzelnen Reaktionsgefäß, wobei das Phenytoin dem Gefäß zugegeben wird, nachdem der Aldehyd oder das Keton, das Lösungsmittel und die Base dem Gefäß zugesetzt wurden, um das 3-(1-Hydroxyalkyl)-5,5-diphenylhydantoin durch Reaktion des Aldehyds oder Ketons und des Phenytoins zu bilden, dadurch gekennzeichnet, daß das Lösungsmittel ein Alkohol ist.

2. Verfahren nach Anspruch 1, wobei die Reaktion bei einer Temperatur von etwa 20°C bis 100°C durchgeführt wird.

3. Verfahren nach Anspruch 2, wobei die Reaktion bei Umgebungstemperatur durchgeführt wird.

4. Verfahren nach irgendeinem vorstehenden Anspruch, wobei die Reaktion über einen Zeitraum von etwa 1 Minute bis etwa 2 Stunden geführt wird.

5. Verfahren nach Anspruch 4, wobei das Verfahren über einen Zeitraum von etwa 15 Minuten geführt wird.

6. Verfahren nach irgendeinem vorstehenden Anspruch, wobei das Lösungsmittel ausgewählt ist aus Methanol, Ethanol, Propanol, Butanol und deren Mischungen.

## Revendications

1. Un procédé de préparation d'un 3-(1-hydroxyalkyl)-5,5-diphényl-hydantoïne de la formule :

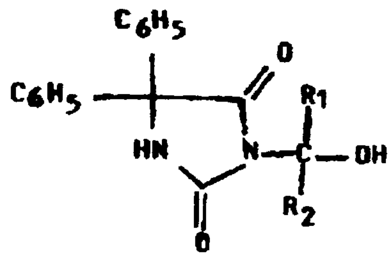

où $R_1$ et $R_2$ sont chacun, de façon indépendante, de l'hydrogène ou un radical alkyle en $C_1$-$C_6$, ou bien $R_1$ et $R_2$ forment ensemble avec l'atome de carbone auquel ils sont liés un groupe cycloalkylène tri- à dodécagonal, selon lequel on mélange, dans un récipient de réaction unique, un aldéhyde ou une cétone de la formule $R_1$ $R_2$-C=O, un solvant, une quantité catalytique d'une base forte minérale, et de la phénytoïne, la phénytoïne étant ajoutée au récipient après que l'aldéhyde ou cétone, le solvant et la base aient été introduits dans le récipient, pour produire le 3-(1-hydroxyalkyl)-5,5-diphénylhydantoïne par réaction de l'aldéhyde ou cétone et de la phénytoïne, caractérisé en ce que le solvant est un alcool.

2. Le procédé de la revendication 1, dans lequel on conduit la réaction à une température d'environ 20° C à 100° C.

3. Le procédé de la revendication 2, dans lequel on conduit la réaction à la température ambiante.

4. Le procédé de l'une quelconque des revendications précédentes, dans lequel on effectue la réaction pendant environ 1 minute à environ 2 heures.

5. Le procédé de la revendication 4, dans lequel on effectue la réaction pendant environ 15 minutes.

6. Le procédé de l'une quelconque des revendications prédédentes, dans lequel on choisit le solvant parmi le méthanol, l'éthanol, le propanol, le butanol et les mélanges de ceux-ci.